# EUROPEAN PATENT APPLICATION

(11) **EP 4 318 335 A1**
(43) Date of publication of application: **07.02.2024**
(21) Application number: 22775724.2
(22) Date of filing: 23.03.2022
(51) Int. Cl.: G06N 20/00, A61B 5/349, A61B 5/352, A61B 5/355, A61B 5/361, A61B 5/366

(54) **ELECTROCARDIOGRAM ANALYSIS ASSISTANCE DEVICE, PROGRAM, ELECTROCARDIOGRAM ANALYSIS ASSISTANCE METHOD, ELECTROCARDIOGRAM ANALYSIS ASSISTANCE SYSTEM, PEAK ESTIMATION MODEL GENERATION METHOD, AND SEGMENT ESTIMATION MODEL GENERATION METHOD**

(30) Priority: 24.03.2021 JP 2021050381
(71) Applicant: Astellas Pharma Inc., Chuo-ku Tokyo 103-8411 (JP)
(72) Inventor: OGINO, Makoto, Tokyo 103-8411 (JP); SHIMIZU, Mitsuhisa, Morioka-shi, Iwate 020-0021 (JP); HASHIMOTO, Hideki, Osaka-shi,Osaka 540-0031 (JP); KAGAWA, Toshiya, Osaka-shi,Osaka 540-0031 (JP)
(74) Representative: V.O.
(86) International application number: PCT/JP2022/013743
(87) International publication number: WO 2022/202942

(57) **Abstract**

An electrocardiogram analysis assistance device (10) including: a peak estimation model (13C) employing waveform data of partial segments of waveform data in an electrocardiogram as input information and employing peak information indicating whether or not a predetermined type of peak related to electrocardiogram analysis is present as output information; an analysis waveform acquisition unit (11C) that acquires waveform data in an electrocardiogram for analysis; an analysis waveform select and extract unit (11D) that selects and extracts waveform data of a predetermined first period from the waveform data acquired by the analysis waveform acquisition unit (11C) as analysis divided waveform data, while each time shifting a predetermined shift period that is a shorter period than the first period; and a derivation unit (11E) that derives the peak information by inputting the analysis divided waveform data selected and extracted by the analysis waveform select and extract unit (11D) into the peak estimation model (13C).

## Description

### Field

The present disclosure relates to an electrocardiogram analysis assistance device, a program, an electrocardiogram analysis assistance method, an electrocardiogram analysis assistance system, a peak estimation model generation method, and a segment estimation model generation method.

### Background Art

Recently measurement data of electrocardiograms has increased dramatically due to proliferation of new electrocardiographic monitors, with this leading to a shortage of labor due to there being hardly any increase in the number of specialists for analyzing such measurement data. Although there are rule-based analysis engines that perform analysis on electrocardiograms, there are limitations to improvements in the performance thereof, and there is often accompanying work for manually correcting the analysis results, with this leading to an increase in the burden on health care professionals who perform such analysis, such as doctors and/or clinical laboratory technicians. There is accordingly a desire for technology capable of reducing the burden on health care professionals.

Hitherto, the following technologies have been available as examples of technology related to electrocardiogram analysis applicable to reducing the burden on health care professionals.

The specification of US Patent Application Laid-Open 2020/0289010 describes an embodiment in which a learning model is generated based on input data related to a training heartbeat waveform generated from a data set including plural items of heartbeat waveform data, with the learning model outputting a heartbeat type of the training heartbeat waveform. The learning model is trained by deciding loss weights for each batch sampled from the data set, and deciding a loss function based on the loss weights for these batches. A test heartbeat waveform is input to the learning model and the test heartbeat waveform is classified into a heartbeat type.

International Publication (WO) No. 2016/092707 discloses a meal intake estimation program that causes the following processing to be executed and is technology for estimating a time when meal intake occurred for the purpose of healthcare including the prevention of life-style diseases such as metabolic syndrome or diabetes, dieting, and/or medical services etc. In a computer, heartrate time series data is acquired and, for each of partial data contained in the heartrate time series data, a feature value is computed related to a second peak appearing following on from a first peak appearing ahead as a peak in heartrate after starting intake of a meal. The feature value related to the second peak computed for each respective partial data is employed to determine whether or not there was meal intake in the partial data, and processing is executed to estimate a meal intake time from the partial data for cases in which meal intake was determined to have occurred.

### SUMMARY OF INVENTION

However, with electrocardiograms it is known that a condition of a peak in waveform data differs according to a type of arrhythmia, such as atrial premature complex, atrial fibrillation, premature ventricular contraction, or the like. It is apparent from results of investigations by the present inventors that whether or not a particular type of arrhythmia has occurred can be determined with high accuracy by analyzing the conditions of such peaks.

However, in the technologies disclosed in the specification of US Patent Application Laid-Open No. 2020/0289010 and in the publication of International Publication (WO) No. 2016/092707, a condition of a peak of waveform data in an electrocardiogram is not considered and there is the issue that a contribution is not always made to determining whether or not a particular type of arrhythmia has occurred.

In consideration of the above circumstances, an object of the present disclosure is to provide an electrocardiogram analysis assistance device and a program that are capable of contributing to determining whether or not a particular type of arrhythmia has occurred.

A first aspect of the present disclosure is an electrocardiogram analysis assistance device including a peak estimation model, an analysis waveform acquisition unit, an analysis waveform select and extract unit, and a derivation unit. The peak estimation model employs waveform data of partial segments of waveform data in an electrocardiogram as input information and employs peak information indicating whether or not a predetermined type of peak related to analysis of the electrocardiogram is present in the waveform data as output information. The analysis waveform acquisition unit acquires waveform data in an electrocardiogram for analysis. The analysis waveform select and extract unit selects and extracts waveform data of a predetermined first period from the waveform data acquired by the analysis waveform acquisition unit as analysis divided waveform data, while each time shifting a predetermined shift period that is a shorter period than the first period. The derivation unit derives the peak information by inputting the analysis divided waveform data selected and extracted by the analysis waveform select and extract unit into the peak estimation model.

A second aspect of the present disclosure is the electrocardiogram analysis assistance device of the first aspect, further including a training waveform acquisition unit that acquires waveform data in electrocardiograms for training and including a training waveform select and extract unit that selects and extracts waveform data of the first period from the waveform data acquired by the training waveform acquisition unit as training divided waveform data. The peak estimation model is a model trained by machine learning employing the training divided waveform data selected and extracted by the training waveform select and extract unit as input information and employing the peak information corresponding to the training divided waveform data as output information.

A third aspect of the present disclosure is the electrocardiogram analysis assistance device of the first aspect or the second aspect, wherein the predetermined type includes at least one type from out of a first type indicting normal or atrial premature complex, a second type indicating atrial fibrillation, and a third type indicating premature ventricular contraction.

A fourth aspect of the present disclosure is the electrocardiogram analysis assistance device of any one of the first aspect to the third aspect, further including a segmentation estimation model, a second analysis waveform select and extract unit, a second derivation unit, and an estimation unit. The segmentation estimation model employs waveform data of partial segments of waveform data in an electrocardiogram as input information and employs segment type information indicating whether or not a segment corresponding to the waveform data is one segment of predetermined types of segment as output information. The second analysis waveform select and extract unit selects and extracts waveform data of a second period that is a longer period than the first period from waveform data acquired by the analysis waveform acquisition unit as second analysis divided waveform data. The second derivation unit derives the segment type information by inputting the second analysis divided waveform data selected and extracted by the second analysis waveform select and extract unit into the segmentation estimation model. The estimation unit estimates a condition indicated by the electrocardiogram for analysis by synthesizing the peak information derived by the derivation unit together with the segment type information derived by the second derivation unit.

A fifth aspect of the present disclosure is the electrocardiogram analysis assistance device of the fourth aspect, wherein the predetermined types of segment include at least two segments from out of a normal segment that is a segment that is normal, an atrial fibrillation segment that is a segment with an atrial fibrillation, and a non-analysis segment that is a segment not for analysis.

A sixth aspect of the present disclosure is a program that causes a computer to execute processing using a peak estimation model employing waveform data of partial segments of waveform data in an electrocardiogram as input information and employing peak information indicating whether or not a predetermined type of peak related to analysis of the electrocardiogram is present in the waveform data as output information. The processing includes acquiring waveform data in an electrocardiogram for analysis, selecting and extracting waveform data of a predetermined first period from the acquired waveform data as analysis divided waveform data while each time shifting a predetermined shift period that is a shorter period than the first period, and deriving the peak information by inputting the selected and extracted analysis divided waveform data into the peak estimation model.

A seventh aspect of the present disclosure is an electrocardiogram analysis assistance method using a peak estimation model employing waveform data of partial segments of waveform data in an electrocardiogram as input information and employing peak information indicating whether or not a predetermined type of peak related to analysis of the electrocardiogram is present in the waveform data as output information. The electrocardiogram analysis assistance method includes acquiring waveform data in an electrocardiogram for analysis, selecting and extracting waveform data of a predetermined first period from the acquired waveform data as analysis divided waveform data while each time shifting a predetermined shift period that is a shorter period than the first period, and deriving the peak information by inputting the selected and extracted analysis divided waveform data into the peak estimation model.

An eighth aspect of the present disclosure is an electrocardiogram analysis assistance system including the electrocardiogram analysis assistance device of any one of the first aspect to the fifth aspect, and a terminal device that transmits waveform data in an electrocardiogram for analysis to the electrocardiogram analysis assistance device and that receives and presents information obtained by the electrocardiogram analysis assistance device in response to the transmission of the waveform data.

A ninth aspect of the present disclosure is a peak estimation model generation method including acquiring first training waveform data in electrocardiograms for training, selecting and extracting waveform data of a predetermined first period from the acquired first training waveform data as first training divided waveform data, and generating a peak estimation model by performing machine learning employing the selected and extracted first training divided waveform data as input information and employing peak information corresponding to the first training divided waveform data and indicating whether or not a predetermined type of peak related to electrocardiogram analysis is present as output information.

A tenth aspect of the present disclosure is a segmentation estimation model generation method including acquiring second training waveform data in electrocardiograms for training, selecting and extracting waveform data of a second period that is a longer period than the predetermined first period from the acquired second training waveform data as second training divided waveform data, and generating a segmentation estimation model by performing machine learning employing the selected and extracted second training divided waveform data as input information and employing segment type information indicating whether or not a segment corresponding to the second training divided waveform data is a segment out of predetermined types of segment as output information.

The present disclosure enables a contribution toward determining whether or not a particular type of arrhythmia has occurred.

### BRIEF DESCRIPTION OF DRAWINGS

Fig. 1 is a block diagram illustrating an example of a hardware configuration of an electrocardiogram analysis assistance device according to one exemplary embodiment.
Fig. 2 is a schematic diagram illustrating an example of a flow of data in a peak estimation model and a segmentation estimation model according to one exemplary embodiment.
Fig. 3 is a block diagram illustrating an example of a functional configuration during training of a peak estimation model and a segmentation estimation model of an electrocardiogram analysis assistance device according to one exemplary embodiment.
Fig. 4 is a block diagram illustrating an example of a functional configuration during operation of a peak estimation model and a segmentation estimation model of an electrocardiogram analysis assistance device according to one exemplary embodiment.
Fig. 5 is a graph to accompany explanation of clustering using a Gaussian mixture model according to one exemplary embodiment.
Fig. 6 is a schematic diagram illustrating an example of a utilization state of middle layers of a peak estimation model according to one exemplary embodiment.
Fig. 7 is a schematic diagram illustrating an example of a configuration of a first training waveform data database according to one exemplary embodiment.
Fig. 8 is a schematic diagram illustrating an example of a configuration of a second training waveform data database according to one exemplary embodiment.
Fig. 9 is a flowchart illustrating an example of training processing according to one exemplary embodiment.
Fig. 10 is a flowchart illustrating an example of electrocardiogram analysis assistance processing according to one exemplary embodiment.
Fig. 11 is a diagram to accompany explanation of electrocardiogram analysis assistance processing according to one exemplary embodiment, and is a waveform diagram illustrating an example of a state in which first analysis divided waveform data has been selected and extracted.
Fig. 12 is a diagram to accompany explanation of electrocardiogram analysis assistance processing according to one exemplary embodiment, and is a waveform diagram illustrating an example of a state in which second analysis divided waveform data has been selected and extracted.
Fig. 13 is a diagram to accompany explanation of electrocardiogram analysis assistance processing according to one exemplary embodiment, and is a schematic diagram illustrating an example of a contracted condition of output from the peak estimation model.
Fig. 14 is a diagram to accompany explanation of electrocardiogram analysis assistance processing according to one exemplary embodiment, and is a schematic diagram illustrating an example of output from a segmentation estimation model.
Fig. 15 is a diagram to accompany explanation of electrocardiogram analysis assistance processing according to one exemplary embodiment, and is a schematic diagram illustrating an example of a synthesized state of output of a peak estimation model together with output of a segmentation estimation model.
Fig. 16 is a diagram to accompany explanation of electrocardiogram analysis assistance processing according to one exemplary embodiment, and is a schematic diagram illustrating an example of a corrected state of a synthesized result of output of a peak estimation model together with output of a segmentation estimation model.
Fig. 17 is a face-on view illustrating an example of a configuration of a result screen displayed during execution of electrocardiogram analysis assistance processing according to one exemplary embodiment.
Fig. 18 is a diagram to accompany explanation of electrocardiogram analysis assistance processing according to one exemplary embodiment, and is a waveform diagram illustrating examples of each type of electrocardiogram waveform represented by classification result information.

### DESCRIPTION OF EMBODIMENTS

Detailed explanation follows regarding an example of an exemplary embodiment of the present disclosure, with reference to the drawings. Note that the same reference numerals will be appended to the same or equivalent configuration elements and parts in each drawing. Moreover, the dimensional proportions of the drawings are sometimes exaggerated for ease of explanation and may differ from actual proportions.

In the present exemplary embodiment, explanation follows regarding an example in which an electrocardiogram analysis assistance device of technology disclosed herein is applied to an information processing device such as a desktop personal computer. However, application targets of technology disclosed herein are not limited to being a desktop information processing device, and application may also be made to a portable information processing device such as a smartphone, a portable game device, a tablet terminal, a notebook personal computer, or the like.

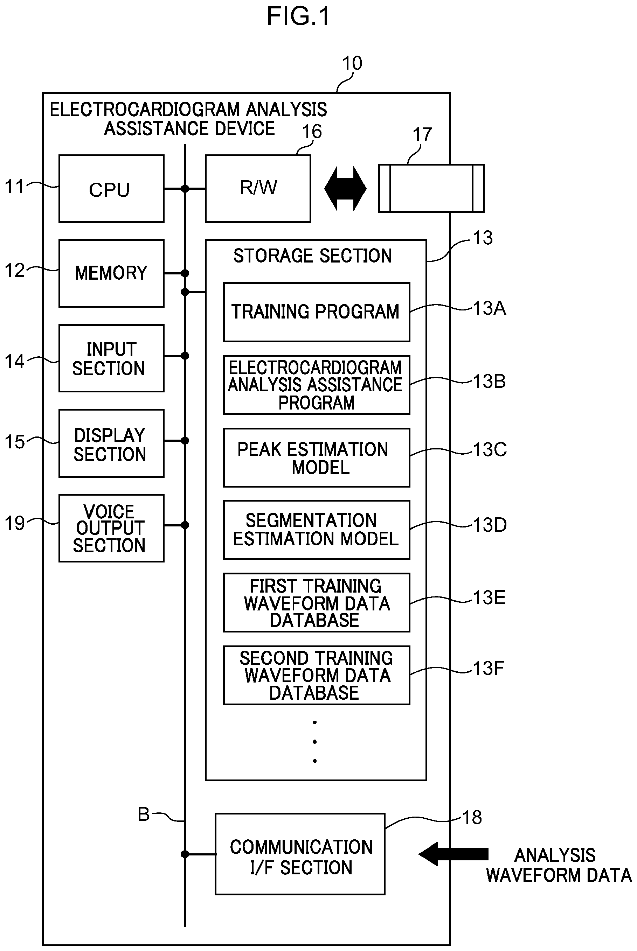

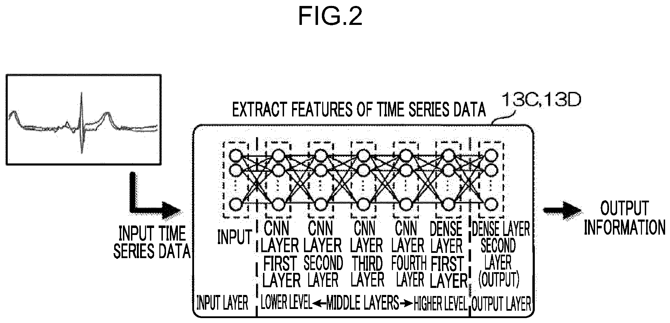

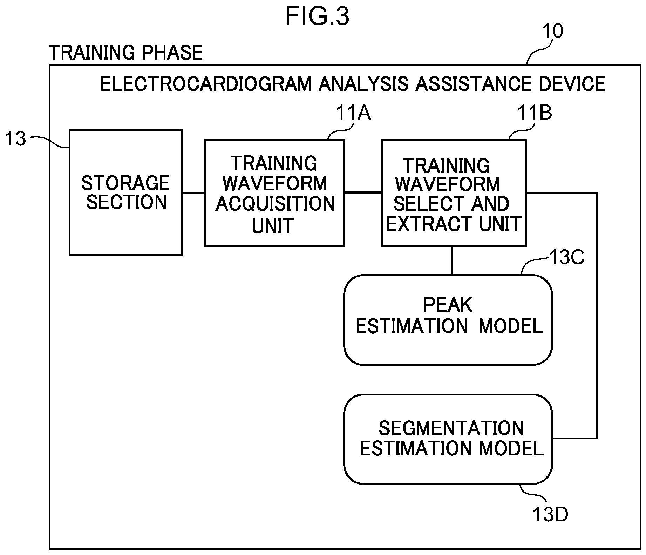

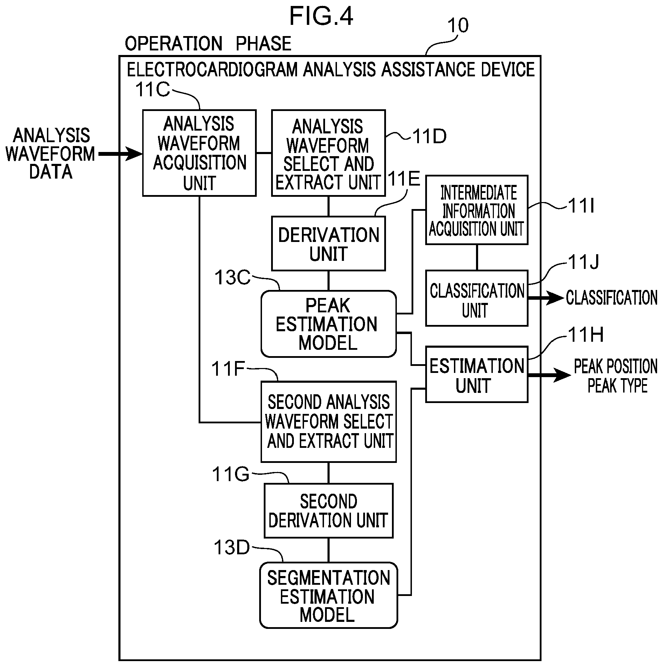

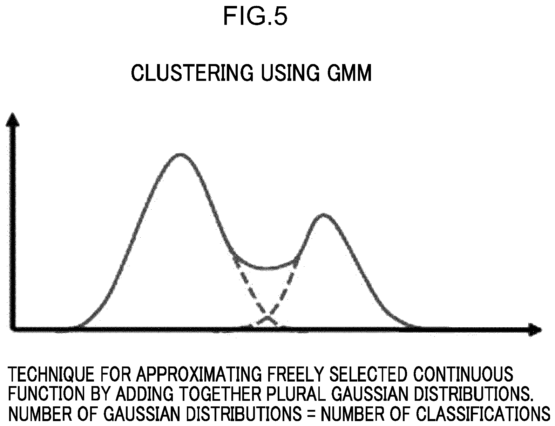

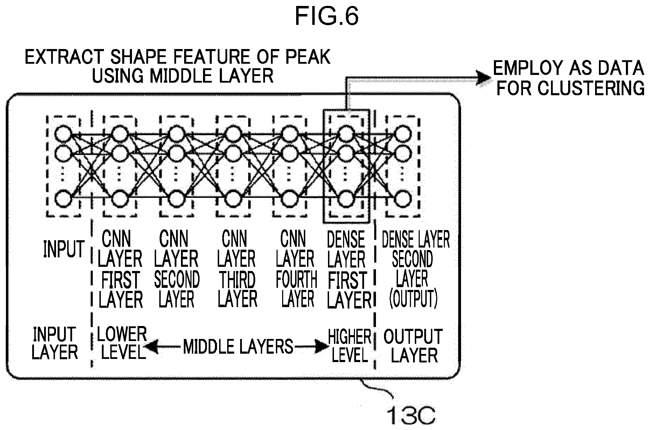

First, description follows regarding a configuration of an electrocardiogram analysis assistance device according to the present exemplary embodiment, with reference to Fig. 1 to Fig. 6. Fig. 1 is a block diagram illustrating an example of a hardware configuration of an electrocardiogram analysis assistance device according to one exemplary embodiment. Fig. 2 is a schematic diagram illustrating an example of a flow of data in a peak estimation model and a segmentation estimation model according to one exemplary embodiment. Fig. 3 is a block diagram illustrating an example of a functional configuration during training of a peak estimation model and a segmentation estimation model of an electrocardiogram analysis assistance device according to one exemplary embodiment. Moreover, Fig. 4 is a block diagram illustrating an example of a functional configuration during operation of a peak estimation model and a segmentation estimation model of an electrocardiogram analysis assistance device according to one exemplary embodiment. Fig. 5 is a graph to accompany explanation of clustering using a Gaussian mixture model according to one exemplary embodiment. Furthermore, Fig. 6 is a schematic diagram illustrating an example of a utilization state of middle layers of a peak estimation model according to one exemplary embodiment.

As illustrated in Fig. 1, an electrocardiogram analysis assistance device 10 according to the present exemplary embodiment includes a central processing unit (CPU) 11, memory 12 serving as a temporary storage area, a non-volatile storage section 13, and an input section 14 such as a keyboard or mouse. The electrocardiogram analysis assistance device 10 according to the present exemplary embodiment also includes a display section 15 such as a liquid crystal display or the like, and a media read/write device (R/W) 16. Furthermore, the electrocardiogram analysis assistance device 10 according to the present exemplary embodiment also includes a communication interface (I/F) section 18, and a voice output section 19. The CPU 11, the memory 12, the storage section 13, the input section 14, the display section 15, and the media read/write device 16, the communication I/F section 18, and the voice output section 19 are each connected together through a bus B. The media read/write device 16 reads information that has been written to a recording medium 17, and writes information to the recording medium 17.

The storage section 13 may be implemented by a hard disk drive (HDD), solid state drive (SSD), flash memory, or the like. A training program 13A and an electrocardiogram analysis assistance program 13B are stored on the storage section 13 serving as a storage medium. The training program 13A is stored on the storage section 13 by the recording medium 17 written with this program 13A being set in the media read/write device 16 and this program 13A being read from the recording medium 17 by the media read/write device 16. The electrocardiogram analysis assistance program 13B is stored in the storage section 13 by the recording medium 17 written with this program 13B being set in the media read/write device 16 and this program 13B being read from the recording medium 17 by the media read/write device 16. The CPU 11 reads the training program 13A and the electrocardiogram analysis assistance program 13B from the storage section 13 and expands these programs into the memory 12, and sequentially executes the processes of the training program 13A and the electrocardiogram analysis assistance program 13B.

Although in the electrocardiogram analysis assistance device 10 according to the present exemplary embodiment the training program 13A and the electrocardiogram analysis assistance program 13B are installed in this manner on the electrocardiogram analysis assistance device 10 via the recording medium 17, there is no limitation thereto. For example, an embodiment may be adopted in which the training program 13A and the electrocardiogram analysis assistance program 13B are installed to the electrocardiogram analysis assistance device 10 by being downloaded via the communication I/F section 18.

A peak estimation model 13C and a segmentation estimation model 13D are also stored on the storage section 13.

The peak estimation model 13C according to the present exemplary embodiment employs waveform data of partial segments of waveform data in an electrocardiogram as input information, and employs peak information indicating whether or not there is a predetermined type of peak related to electrocardiogram analysis present in the waveform data as output information.

Note that although in the present exemplary embodiment a configuration is adopted in which the predetermined types include all of a first type indicting normal or atrial premature complex, a second type indicating atrial fibrillation, and a third type indicating premature ventricular contraction, there is no limitation thereto. For example, a combination of one type or two types from out of these three types may be included as the predetermined types, or another type of arrhythmia other than these three types may be may be added for inclusion in the predetermined types. In the following the first type is expressed as "N or S" or "N", the second type is expressed as "small_n" or "n", and the third type is expressed as "PVC" or "V". Moreover, in the following, a type of no presence of the predetermined peak types is expressed as "FALSE".

Moreover in the present exemplary embodiment, the peak information not only includes information indicating whether or not the predetermined types of peak related to electrocardiogram analysis are present in the waveform data, but if such a peak is present then also includes information indicating the predetermined type when such as peak is present. Thus in addition to the peak estimation model 13C according to the present exemplary embodiment outputting the above "FALSE", when not "FALSE", namely when such a peak is present, the peak estimation model 13C is configured so as to also outputs information indicating the type of arrhythmia, such as "N or S" etc., as the peak information.

The segmentation estimation model 13D according to the present exemplary embodiment employs waveform data of partial segments of waveform data in an electrocardiogram as input information, and employs segment type information indicating which segment the segment corresponding to the waveform data is from out of predetermined types of segment as output information.

Note that although in the present exemplary embodiment the predetermined types of segment include all segments of a normal segment that is a segment that is normal, an atrial fibrillation segment that is a segment with an atrial fibrillation, and a non-analysis segment that is a segment not for analysis, there is no limitation thereto. For example, a combination of two types from out of these three types may be included in the predetermined types of segment, or another segment other than these three types may be added for inclusion in the predetermined types of segment.

Although in the present exemplary embodiment a convolutional neural network (CNN) is employed as the peak estimation model 13C and the segmentation estimation model 13D, there is no limitation thereto. For example, a configuration may be adopted in which a recurrent neural network (RNN) is employed for at least one out of the peak estimation model 13C or the segmentation estimation model 13D.

As in the example illustrated in Fig. 2, in the present exemplary embodiment a onedimensional CNN employed as the peak estimation model 13C and the segmentation estimation model 13D includes four layers of CNN layers that are configured including convolutional/pooling layers and two layers of dense layers.

A first training waveform data database 13E and a second training waveform data database 13F are stored in the storage section 13. A detailed description is given later regarding the first training waveform data database 13E and the second training waveform data database 13F.

Next, description follows regarding a functional configuration during training of the peak estimation model 13C and the segmentation estimation model 13D of the electrocardiogram analysis assistance device 10 according to the present exemplary embodiment, with reference to Fig. 3.

As illustrated in Fig. 3, during training of the peak estimation model 13C and the segmentation estimation model 13D, the electrocardiogram analysis assistance device 10 includes a training waveform acquisition unit 11A and a training waveform select and extract unit 11B. The CPU 11 of the electrocardiogram analysis assistance device 10 functions as the training waveform acquisition unit 11A and the training waveform select and extract unit11B by executing the training program 13A.

The training waveform acquisition unit 11A according to the present exemplary embodiment acquires waveform data in an electrocardiogram for training (hereafter referred to as "training waveform data") for each of the models of the peak estimation model 13C and the segmentation estimation model 13D.

Note that in the present exemplary embodiment training waveform data for the peak estimation model 13C (hereinafter referred to as "first training waveform data") is acquired by reading from the first training waveform data database 13E, described later. Moreover, in the present exemplary embodiment the training waveform data (hereinafter referred to as "second training waveform data") for the segmentation estimation model 13D is acquired by reading from the second training waveform data database 13F, described later. However, there is no limitation to such an embodiment, and an embodiment may be adopted in which waveform data obtained by an electrocardiographic monitor is directly acquired as training waveform data via the communication I/F section 18 or the like.

Moreover, from the first training waveform data acquired by the training waveform acquisition unit 11A, the training waveform select and extract unit11B according to the present exemplary embodiment selects and extracts waveform data of a predetermined first period (hereinafter simply referred to as "first period") as training divided waveform data (hereafter referred to as "first training divided waveform data").

Note that in the training waveform select and extract unit 11B according to the present exemplary embodiment, selecting and extracting of the first training divided waveform data is performed by selecting and extracting waveform data of the first period centered on a time axis center of the first training waveform data, however, there is no limitation thereto. For example, an embodiment may be adopted in which a time axis leading end of the first training waveform data is selected and extracted as a leading end for the waveform data of the first period, or an embodiment may be adopted in which a time axis trailing end of the first training waveform data is selected and extracted as a trailing end for the waveform data of the first period.

Moreover, in the present exemplary embodiment, although two seconds is employed for the first period, there is no limitation thereto. For example, an embodiment may be adopted in which a period other than two seconds, such as 1.8 seconds, 2.5 seconds, or the like is employed as the first period. Employing two seconds as the first period is because the interval of general peaks in an electrocardiogram of about one second, and so this enables information of the peak and before and after the peak to be obtained appropriately when set to about two seconds.

Moreover from the second training waveform data acquired by the training waveform acquisition unit 11A, the training waveform select and extract unit 11B according to the present exemplary embodiment selects and extracts waveform data of a second period that is a period longer than the first period (hereinafter simply referred to as a "second period") as training divided waveform data (hereafter referred to as "second training divided waveform data").

Note that although in the training waveform select and extract unit 11B according to the present exemplary embodiment the selection and extraction of the second training divided waveform data is performed by selecting and extracting waveform data of the second period centered on a time axis center of the second training waveform data, there is no limitation thereto. For example, an embodiment may be adopted in which a time axis leading end of the second training waveform data is selected and extracted as a leading end for the waveform data of the second period, or an embodiment may be adopted in which a time axis trailing end of the second training waveform data is selected and extracted as a trailing end for the waveform data of the second period.

Moreover, although five seconds is employed as the second period in the present exemplary embodiment, there is no limitation thereto. For example, an embodiment may be adopted in which a period other than five seconds, such as 4.8 seconds or 5.5 seconds is employed as the second period. Employing five seconds as the second period is because, as a result of trial and error by the present inventors, it has been found that by setting the second period to about five seconds a result is obtained that enables sufficient information to be obtained to determine an atrial fibrillation segment and a non-analysis segment, while being able to suppress mixed presence of atrial fibrillation segments and non-analysis segments.

Machine learning is performed on the peak estimation model 13C according to the present exemplary embodiment using the first training divided waveform data selected and extracted by the training waveform select and extract unit 11B as input information, and with the above peak information corresponding to the first training divided waveform data as output information. Moreover, machine learning is performed on the segmentation estimation model 13D according to the present exemplary embodiment employing the second training divided waveform data selected and extracted by the training waveform select and extract unit 11B as input information, and employing the above segment type information corresponding to the second training divided waveform data as output information.

Next, description follows regarding a functional configuration of the electrocardiogram analysis assistance device 10 according to the present exemplary embodiment during operation of the peak estimation model 13C and the segmentation estimation model 13D, with reference to Fig. 4.

As illustrated in Fig. 4, the electrocardiogram analysis assistance device 10 during operation of the peak estimation model 13C and the segmentation estimation model 13D includes an analysis waveform acquisition unit 11C, an analysis waveform select and extract unit 11D, a derivation unit 11E, a second analysis waveform select and extract unit 11F, a second derivation unit 11G, an estimation unit 11H, an intermediate information acquisition unit 111, and a classification unit 1 1J. The CPU 11 of the electrocardiogram analysis assistance device 10 functions as the analysis waveform acquisition unit 11C, the analysis waveform select and extract unit 11D, the derivation unit 11E, the second analysis waveform select and extract unit 11F, the second derivation unit 11G, the estimation unit 11H, the intermediate information acquisition unit 111, and the classification unit 1 1J by executing the electrocardiogram analysis assistance program 13B.

The analysis waveform acquisition unit 11C according to the present exemplary embodiment acquires waveform data in an electrocardiogram for analysis (hereafter referred to as "analysis waveform data"). Note that in the present exemplary embodiment an embodiment is adopted in which the analysis waveform data is directly acquired from an electrocardiographic monitor via the communication I/F section 18 or the like. However, there is no limitation to such an embodiment, and an embodiment may be adopted in which waveform data of electrocardiograms for analysis are placed in a database, and the analysis waveform data is acquired by reading from the database.

Moreover from the analysis waveform data acquired by the analysis waveform acquisition unit 11C, the analysis waveform select and extract unit 11D according to the present exemplary embodiment selects and extracts waveform data of the first period as the first analysis divided waveform data while each time moving a predetermined shift period that is a period shorter than the first period (hereinafter simply referred to as "shift period").

Note that although in the present exemplary embodiment 0.1 seconds is employed as the shift period, there is no limitation thereto. For example, an embodiment may be adopted in which another period shorter than the first period, such as 0.05 seconds or 0.2 seconds, is employed as the shift period. Employing 0.1 seconds as the shift period is because, as a result of trial and error by the present inventors, it has been found that by setting the shift period to about 0.1 seconds, a result is obtained that enables non-detection of peaks in the electrocardiogram to be prevented, while also enabling computation time to be prevented from being prolonged.

The derivation unit 11E according to the present exemplary embodiment uses the first analysis divided waveform data selected and extracted by the analysis waveform select and extract unit 11D as input to the peak estimation model 13C to derive the peak information using the peak estimation model 13C.

From the analysis waveform data acquired by the analysis waveform acquisition unit 11C, the second analysis waveform select and extract unit 11F according to the present exemplary embodiment selects and extracts waveform data of the second period as the second analysis divided waveform data. Moreover, the second derivation unit 11G according to the present exemplary embodiment derives the above segment type information using the segmentation estimation model 13D using the second analysis divided waveform data selected and extracted by the second analysis waveform select and extract unit 11F as input to the segmentation estimation model 13D.

The estimation unit 11H according to the present exemplary embodiment estimates a condition indicted by the electrocardiogram for analysis by synthesizing the peak information derived by the derivation unit 11E using the peak estimation model 13C input with the first analysis divided waveform data, together with the segment type information derived by the second derivation unit 11G using the segmentation estimation model 13D input with the second analysis divided waveform data.

By inputting the first analysis divided waveform data selected and extracted by the analysis waveform select and extract unit 11D into the peak estimation model 13C, the intermediate information acquisition unit 111 according to the present exemplary embodiment acquires intermediate information generated in a middle layer of the peak estimation model 13C that indicates a shape feature of a peak contained in the first analysis divided waveform data (hereinafter simply referred to as "intermediate information").

The classification unit 11J according to the present exemplary embodiment classifies a type of the waveform of the electrocardiogram for analysis using the intermediate information acquired by the intermediate information acquisition unit 111.

Note that although in the present exemplary embodiment all types of a standard type, a type having peaked T waves taller than the standard type, a type having wide QRS waves wider than the standard type, and an other-type therefrom are included as the above waveform types, there is no limitation thereto. For example, a combination of two types or three types from out of these four types may be included in the above waveform types, or another fourth type may be added for inclusion in the above waveform types.

Moreover, the classification unit 11J according to the present exemplary embodiment is an embodiment configured to perform the above classification by clustering the intermediate information.

Note that in the present exemplary embodiment clustering using a Gaussian mixture model (GMM) such as illustrated in the example in Fig. 5 is employed for such clustering. Clustering using a Gaussian mixture model is a technique for approximating a freely selected continuous function by adding together plural Gaussian distributions, with the number of Gaussian distributions being the number of classifications. However, the above clustering is not limited to clustering using a Gaussian mixture model and, for example, an embodiment may be adopted in which the above classification is performed using another clustering technique, such as spectral clustering.

As illustrated in the example in Fig. 6, in the electrocardiogram analysis assistance device 10 according to the present exemplary embodiment, the data generated by the first layer of the dense layers in the peak estimation model 13C is employed as the intermediate information, and this data is employed for clustering.

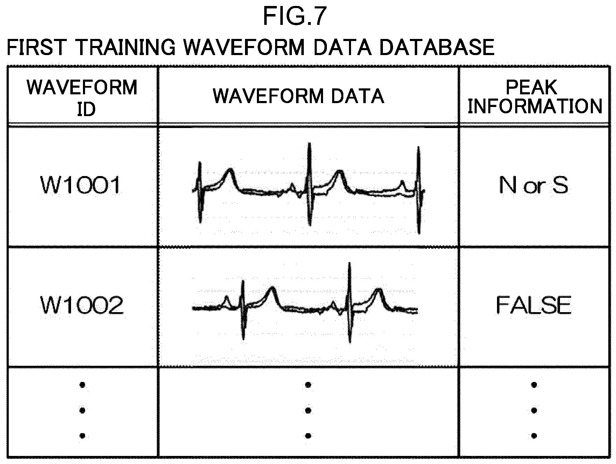

Next, description follows regarding the first training waveform data database 13E according to the present exemplary embodiment, with reference to Fig. 7. Fig. 7 is a schematic diagram illustrating an example of a configuration of the first training waveform data database 13E according to one exemplary embodiment.

The first training waveform data database 13E according to the present exemplary embodiment is a database for storing information related to the first training waveform data used for training the peak estimation model 13C as described above.

As illustrated in Fig. 7, the first training waveform data database 13E according to the present exemplary embodiment is stored with each information of a waveform identification (ID), waveform data, and peak information.

The waveform ID is employed to discriminate between corresponding waveform data, and is information pre-assigned so as to be different for each item of the waveform data. The above waveform data is information representing the first training waveform data itself, and the above peak information is information indicating correct peak information (correct information) that should be output from the peak estimation model 13C when the corresponding waveform data has been input.

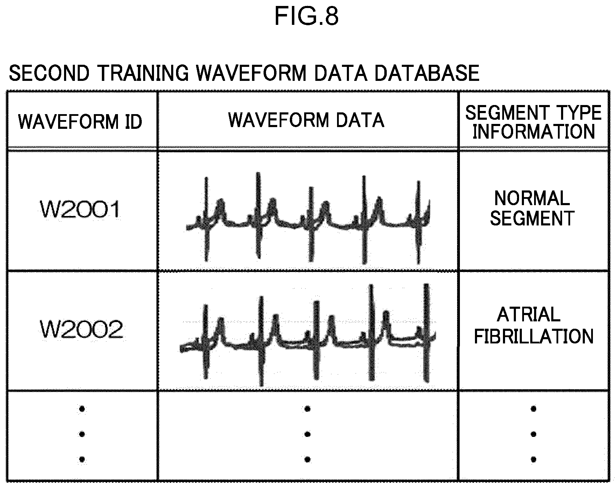

Next, description follows regarding the second training waveform data database 13F according to the present exemplary embodiment, with reference to Fig. 8. Fig. 8 is a schematic diagram illustrating an example of a configuration of the second training waveform data database 13F according to one exemplary embodiment.

The second training waveform data database 13F according to the present exemplary embodiment is a database stored with information related to the second training waveform data employed to train the segmentation estimation model 13D as described above.

As illustrated in Fig. 8, the second training waveform data database 13F according to the present exemplary embodiment is stored with each information of waveform ID, waveform data, and segment type information.

The waveform ID is employed to discriminate between corresponding waveform data, and is information pre-assigned so as to be different for each item of the waveform data. The above waveform data is information representing the second training waveform data itself, and the segment type information is information indicating correct segment type information (correct information) that should be output from the segmentation estimation model 13D when the corresponding waveform data has been input.

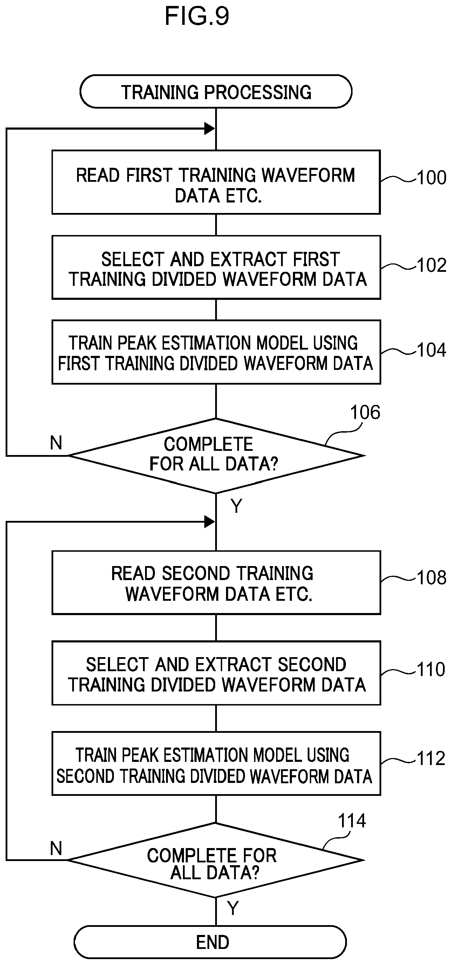

Next, description follows regarding operation of the electrocardiogram analysis assistance device 10 according to the present exemplary embodiment, with reference to Fig. 9 to Fig. 18. First, description follows regarding operation of the electrocardiogram analysis assistance device 10 when training the peak estimation model 13C and the segmentation estimation model 13D, with reference to Fig. 9. Fig. 9 is a flowchart illustrating an example of training processing according to one exemplary embodiment.

The training processing illustrated in Fig. 9 is executed by the CPU 11 of the electrocardiogram analysis assistance device 10 executing the training program 13A. The training processing illustrated in Fig. 9 is executed when an instruction input to start execution of the training program 13A is performed by a user through the input section 14. In order to avoid confusion, the following describes a case in which the first training waveform data database 13E and the second training waveform data database 13F have already been built.

At step 100 of Fig. 9, the CPU 11 reads a pair of waveform data (first training waveform data) and peak information from the first training waveform data database 13E. At step 102, the CPU 11 selects and extracts the first training divided waveform data from the read first training waveform data as described above.

At step 104, the CPU 11 performs machine learning on the peak estimation model 13C employing the selected and extracted first training divided waveform data as input information and employing the read peak information as output information (correct information). At step 106, the CPU 11 determines whether or not the machine learning of step 104 has been completed for all waveform data stored in the first training waveform data database 13E, with processing returning to step 100 when negative determination is made, and processing transitioning to step 108 when affirmative determination is made. Note that in cases in which the processing of step 100 to step 106 is being executed again, the CPU 11 uses first training waveform data that has not previously been subjected to processing as the processing target.

At step 108, the CPU 11 reads a pair of the waveform data (second training waveform data) and segment type information from the second training waveform data database 13F. At step 110, the CPU 11 selects and extracts the second training divided waveform data from the read second training waveform data as described above.

At step 112, the CPU 11 performs machine learning on the segmentation estimation model 13D employing the selected and extracted second training divided waveform data as input information and employing the read segment type information as output information (correct information). At step 114, the CPU 11 determines whether or not the machine learning of step 112 has been completed for all waveform data stored in the second training waveform data database 13F, with processing returning to step 108 when negative determination is made, and the present training processing ending when affirmative determination is made. Note that in cases in which the processing of step 108 to step 114 is being executed again, the CPU 11 uses second training waveform data that has not previously been subjected to processing as the processing target.

The peak estimation model 13C and the segmentation estimation model 13D are trained by the training processing described above. Note that an embodiment may be adopted in which similar training processing is executed again in cases such as when estimation accuracy of the peak estimation model 13C and the segmentation estimation model 13D as obtained by the above training processing is not sufficient.

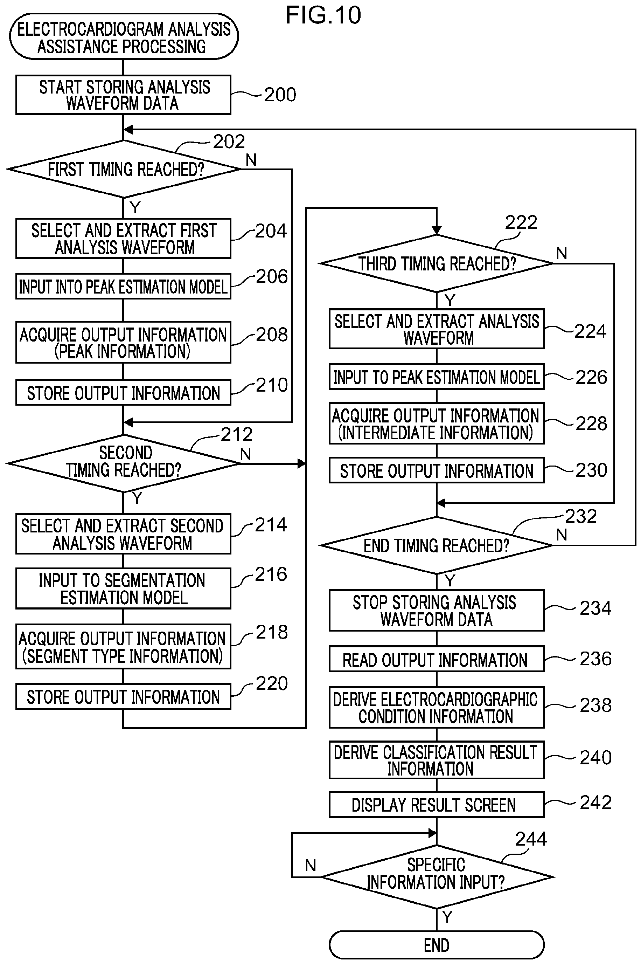

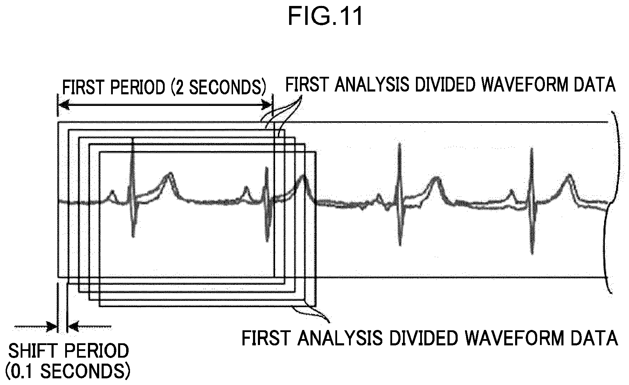

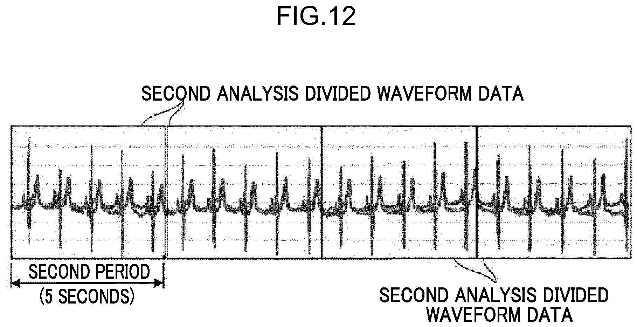

Next, description follows regarding operation of the electrocardiogram analysis assistance device 10 when the peak estimation model 13C and the segmentation estimation model 13D are being operated, with reference to Fig. 10 to Fig. 18. Fig. 10, is a flowchart illustrating an example of electrocardiogram analysis assistance processing according to one exemplary embodiment. Fig. 11 is a diagram to accompany explanation of electrocardiogram analysis assistance processing according to one exemplary embodiment, and is a waveform diagram illustrating an example of a state in which first analysis divided waveform data has been selected and extracted. Furthermore, Fig. 12 is a diagram to accompany explanation of electrocardiogram analysis assistance processing according to one exemplary embodiment, and is a waveform diagram illustrating an example of a state in which second analysis divided waveform data has been selected and extracted.

The electrocardiogram analysis assistance processing illustrated in Fig. 10 is executed by the CPU 11 of the electrocardiogram analysis assistance device 10 executing the electrocardiogram analysis assistance program 13B. The electrocardiogram analysis assistance processing illustrated in Fig. 10 is executed when an instruction input to start execution of the electrocardiogram analysis assistance program 13B is performed by a user through the input section 14. In order to avoid confusion, the following describes a case in which training of the peak estimation model 13C and the segmentation estimation model 13D is complete. Moreover, description follows regarding an example of a case in which a nonillustrated electrocardiographic monitor that has been worn by a person subject to electrocardiogram analysis is connected to the communication I/F section 18 of the electrocardiogram analysis assistance device 10, and a state has been achieved in which waveform data in an electrocardiogram from the electrocardiographic monitor is receivable by the electrocardiogram analysis assistance device 10.

At step 200 of Fig. 10, the CPU 11 starts storing waveform data received from the electrocardiogram (corresponding to the analysis waveform data described above, and hereafter referred to as "analysis waveform data") in the storage section 13. At step 202, the CPU 11 determines whether or not a predetermined timing (hereafter referred to as a "first timing") has been reached as a timing to perform estimation using the peak estimation model 13C, and processing transitions to step 212 in cases in which negative determination is made. The CPU 11 transitions to step 204 when affirmative determination is made at step 202. Note that in the present exemplary embodiment a timing of when the first period has ended, using a predetermined reference time (a time when execution of the present electrocardiogram analysis assistance processing is started in the present exemplary embodiment) as a start time, and the shift period has ended may be employed as the first timing.

At step 204, the CPU 11 selects and extracts the first analysis divided waveform data by reading a first period's worth of the analysis waveform data from the storage section 13. At step 206, the CPU 11 inputs the selected and extracted first analysis divided waveform data into the peak estimation model 13C, and at step 208, the CPU 11 acquires peak information output from the peak estimation model 13C in response to input of the first analysis divided waveform data. At step 210, the CPU 11 stores the acquired peak information in the storage section 13.

By repeatedly executing the processing of step 202 to step 210 as described above, as illustrated in the example in Fig. 11, the first analysis divided waveform data having a width of the first period (two seconds in the present exemplary embodiment) is input into the peak estimation model 13C at a step width of the shift period (0.1 seconds in the present exemplary embodiment), and the peak information output from the peak estimation model 13C in response thereto is stored in the storage section 13.

At step 212, the CPU 11 determines whether or not a predetermined timing has been reached as a timing to perform estimation using the segmentation estimation model 13D (hereafter referred to as a "second timing"), and processing transitions to step 222 in cases in which negative determination is made. The CPU 11 transitions to step 214 in cases in which affirmative determination has been made at step 212. Note that in the present exemplary embodiment a timing when the second period has ended using the reference time as the origin is employed as the above second timing.

At step 214, the CPU 11 selects and extracts the second analysis divided waveform data by reading the second period's worth of the analysis waveform data from the storage section 13. At step 216, the CPU 11 inputs the second analysis divided waveform data that was selected and extracted to the segmentation estimation model 13D, and at step 218, the CPU 11 acquires segment type information output from the segmentation estimation model 13D in response to input of the second analysis divided waveform data. At step 220, the CPU 11 stores the acquired segment type information in the storage section 13.

By repeatedly executing the processing of step 212 to step 220 as described above, as illustrated in the example in Fig. 12, the second analysis divided waveform data having a width of the second period (five seconds in the present exemplary embodiment) is input to the segmentation estimation model 13D, and the segment type information output from the segmentation estimation model 13D in response thereto is stored in the storage section 13.

At step 222, the CPU 11 determines whether or not a predetermined timing has been reached as a timing to acquire intermediate information for clustering by the classification unit 1 1J (hereafter referred to as a "third timing"), and processing transitions to step 232 in cases in which negative determination is made. The CPU 11 transitions to step 224 in cases in which affirmative determination has been made at step 222. Note that in the present exemplary embodiment a timing when peak information other than "FALSE" was stored by the processing of step 210, namely a timing when any peak was detected in the analysis waveform data, may be employed as the above third timing.

At step 224, the CPU 11 selects and extracts the analysis divided waveform data by reading the first period's worth of the analysis waveform data from the storage section 13. At step 226, the CPU 11 inputs the selected and extracted analysis divided waveform data into the peak estimation model 13C, and at step 228, the CPU 11 acquires intermediate information generated in a middle layer (the first layer of the dense layers in the present exemplary embodiment) of the peak estimation model 13C in response to input of the analysis divided waveform data. At step 230, the CPU 11 stores the acquired intermediate information in the storage section 13.

At step 232, the CPU 11 determines whether or not a predetermined timing to end measurement (hereafter referred to as an "end timing") has been reached, with processing returning to step 202 when negative determination is made, and processing transitioning to step 234 when affirmative determination is made. Note that although in the present exemplary embodiment a timing when a number of peaks occurring in the analysis waveform data from the reference time has reached a predetermined threshold (60 in the present exemplary embodiment) is employed as the above end timing, there is no limitation thereto. For example, an embodiment may be adopted in which a timing when a predetermined period (60 seconds in the present exemplary embodiment) has elapsed from the reference time is employed as the above end timing.

At step 234, the CPU 11 stops the storing of the analysis waveform data in the storage section 13 that was started by the processing of step 200. At step 236, the CPU 11 reads all of the peak information, the segment type information, and the intermediate information stored by the above processing from the storage section 13.

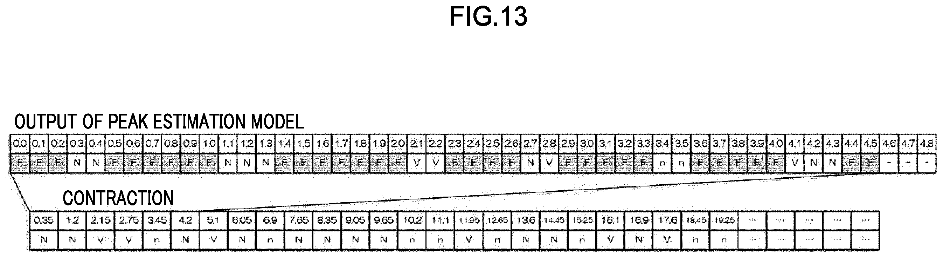

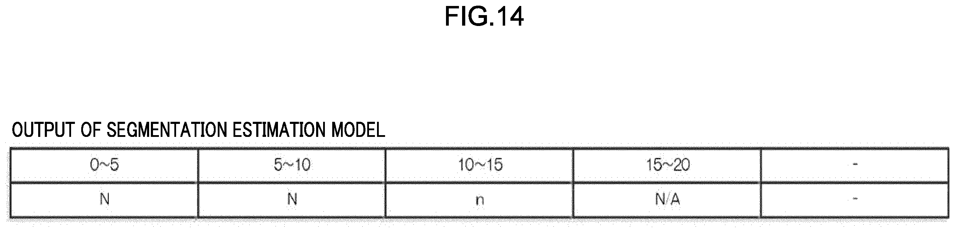

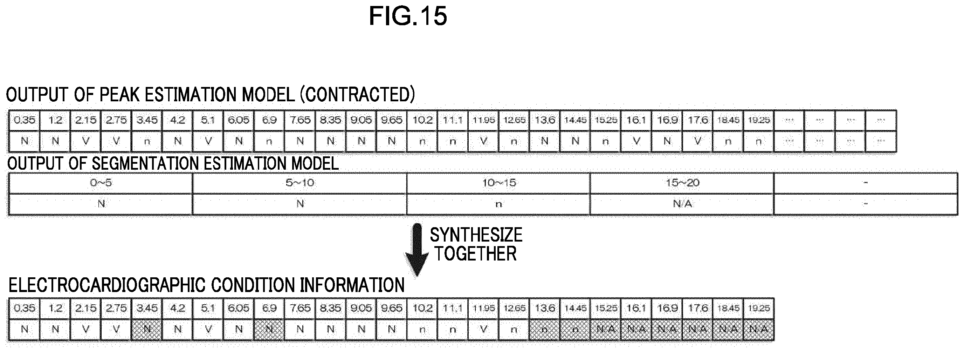

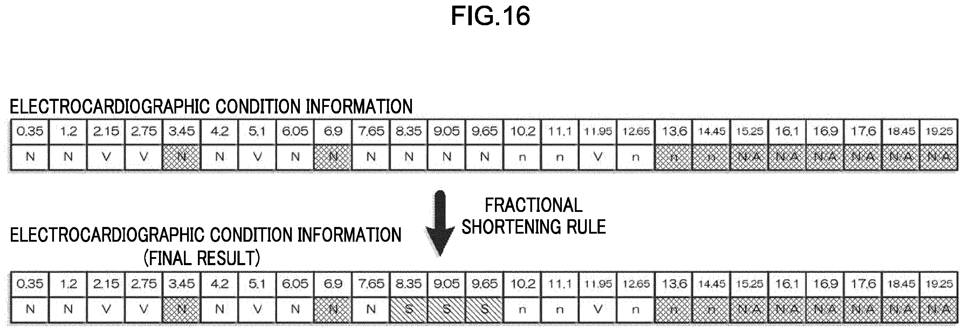

At step 238, the CPU 11 employs the read peak information and segment type information to estimate a condition indicted by the electrocardiogram for analysis. Description follows regarding such estimation, with reference to Fig. 13 to Fig. 16. Note that Fig. 13 is a diagram to accompany explanation of electrocardiogram analysis assistance processing according to one exemplary embodiment, and is a schematic diagram illustrating an example of a contracted condition of output from the peak estimation model. Fig. 14 is a diagram to accompany explanation of electrocardiogram analysis assistance processing according to one exemplary embodiment, and is a schematic diagram illustrating an example of output from a segmentation estimation model. Fig. 15 is a diagram to accompany explanation of electrocardiogram analysis assistance processing according to one exemplary embodiment, and is a schematic diagram illustrating an example of a synthesized state of output of a peak estimation model and output of a segmentation estimation model. Furthermore, Fig. 16 is a diagram to accompany explanation of electrocardiogram analysis assistance processing according to one exemplary embodiment, and is a schematic diagram illustrating an example of a corrected state of a synthesized result of output of a peak estimation model together with output of a segmentation estimation model.

The upper layer of each of the double row configurations in Fig. 13 to Fig. 16 represents the passage of time, and the lower layer therein represent the corresponding output information from the model. Moreover, in Fig. 13 to Fig. 16, in the peak information output from the peak estimation model 13C "FALSE" is indicated by "F", "N or S" is indicated by "N", "PVC" is indicated by "V", and "small_n" is indicated by "n". Furthermore, in Fig. 14 to Fig. 16, in the segment type information output from the segmentation estimation model 13D a normal segment is indicated by "N", an atrial fibrillation segment is indicated by "n", and a non-analysis segment is indicated by "N/A" (no analyze).

First, according to predetermined rules the CPU 11 contracts the read peak information into only information indicating a peak position and a type of peak. Note that the following rules are employed in the present exemplary embodiment as the above rules.

Rule 1 regarding peak position: for a single isolated peak a position of the peak is employed, and for plural successive peaks a center position of the group of peaks is employed.

Rule 2 regarding type of peak: for a single isolated peak a type of the peak is employed, and for plural successive peaks a most frequent type of the types of the group of peaks is employed. When doing so in situations in which there are plural types present with the highest frequency, then in such cases the type of peak is decided in a priority sequence of "PVC" → "N or S" → "small_n".

Note that the following are the reason for using the above priority sequence.

Namely, there is a need to make the possibility of overlooking a premature ventricular contraction (PVC) as small as possible. The priority of premature ventricular contraction V is accordingly set to the highest based on the consideration that a determination of premature ventricular contraction V should be made if there is any possibility of there being a premature ventricular contraction V.

However, whether or not there is an atrial fibrillation n (small_n) is ultimately determined based on results of the segmentation estimation model 13D, and so determination of atrial fibrillation n by the peak estimation model 13C is merely a complementary determination. The priority of atrial fibrillation n is accordingly set to the lowest priority.

A contracted result by such processing as illustrated in the lower level of Fig. 13 is for the read peak information of the upper level of Fig. 13.

Next, the CPU 11 synthesizes the contracted peak information together with the read segment type information. When this is performed, the CPU 11 synthesizes the peak information together with the segment type information in the following manner.

Firstly, the CPU 11 corrects a peak of "small_n" falling within a segment determined to be a normal segment N by the segmentation estimation model 13D by correction to "N or S".

Secondly, the CPU 11 corrects a peak of "N or S" within a segment determined to be an atrial fibrillation segment n by the segmentation estimation model 13D to "small_n".

Thirdly, the CPU 11 takes a peak within a segment determined to be a non-analysis segment N/A by the segmentation estimation model 13D as being misdetection of noise, and excludes all these from the peaks. (However, these are retained internally).

Fig. 13 illustrates information output from the peak estimation model 13C on which the contraction described above has been performed, and Fig. 15 illustrates a result of performing the above synthesis for a case in which the segment type information output from the segmentation estimation model 13D is as illustrated in Fig. 14.

In this case the peaks at 3.45 seconds and 6.9 seconds have been corrected from "small_n" to "N or S". The peaks at 13.6 seconds and 14.45 seconds have been corrected from "N or S" to "small_n".Furthermore, the peaks from 15.25 seconds onward are excluded from the peaks.

Data after synthesis illustrated as an example at the lower level of Fig. 15 is data representing a condition indicted by the electrocardiogram for analysis obtained by the estimation unit 11H, and this data is hereafter referred to as "electrocardiographic condition information".

Finally, the CPU 11 corrects the electrocardiographic condition information by performing determination to allocate "N or S" output from the peak estimation model 13C to normal N and to atrial premature complex APC using similarly fractional shortening rules to those of known rule-based analysis. Description follows regarding fractional shortening rules according to the present exemplary embodiment.

Namely, although normal N and atrial premature complex APC do not greatly differ in waveforms themselves, peaks of normal N appear regularly, whereas peak of atrial premature complex APC appear at an earlier timing than normal. This means that with a rule-based algorithm atrial premature complex APC can be determined when an interval to an immediately previous peak is shorter than normal. More specifically, atrial premature complex APC is determined when the interval to an immediately previous peak is 0.8 times a moving average or shorter. This 0.8 times threshold is called a "fractional shortening".

The final electrocardiographic condition information obtained by such correction is as illustrated as an example in Fig. 16.

At step 240, the CPU 11 derives classification result information indicating results in which a type of the electrocardiogram for analysis has been classified by performing clustering on the read intermediate information as described above (clustering using a GMM in the present exemplary embodiment).

At step 242, the CPU 11 controls the display section 15 using the electrocardiographic condition information and the classification result information derived by the processing described above so as to display a result screen of a predetermined configuration, and at step 244 the CPU 11 stands by for input of specific information.

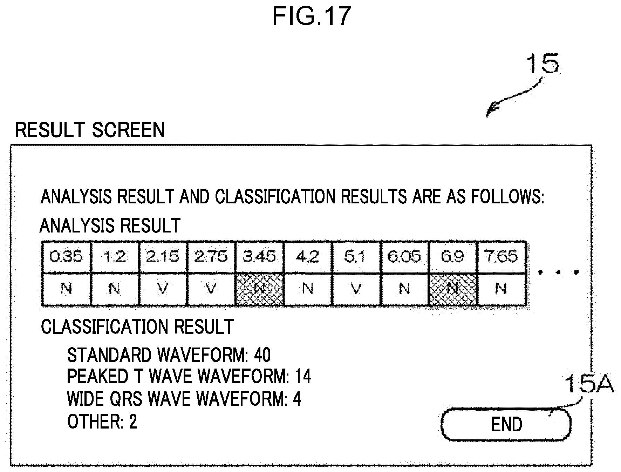

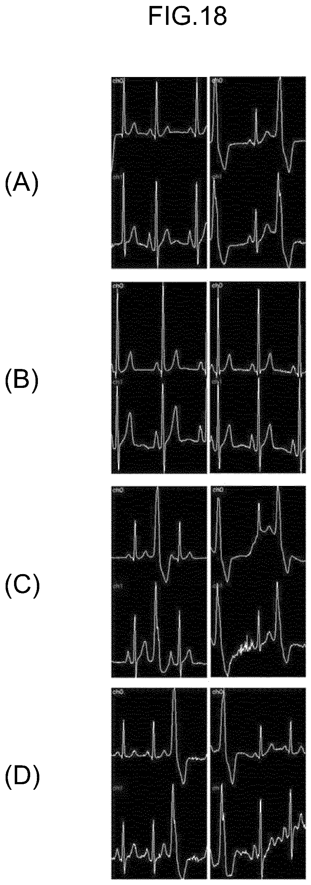

An example of a result screen according to the present exemplary embodiment is illustrated in Fig. 17. As illustrated in Fig. 17, the result screen according to the present exemplary embodiment displays information indicating a class of each peak of the electrocardiogram for analysis, a position of each peak, and classification results. This thereby enables a user to ascertain each such information by referencing the result screen. Note that Fig. 18 is a waveform diagram illustrating examples of each type of electrocardiogram waveform illustrated by the classification result information. (A) in Fig. 18 illustrates an example of a waveform belonging to a standard type, and (B) therein illustrates an example of a waveform belonging to a type having peaked T waves taller than the standard type. (C) in Fig. 18 illustrates an example of a waveform belonging to a type having wide QRS waves wider than the standard type, and (D) therein is an example of a waveform of an other-type therefrom.

When the result screen as illustrated as an example in Fig. 17 is displayed on the display section 15, the user selects an end button 15A through the input section 14 after ascertaining the content being displayed. When the end button 15A has been selected by the user, the current electrocardiogram analysis assistance processing is ended when affirmative determination is made at step 244.

As described above, one exemplary embodiment includes a peak estimation model 13C employing waveform data of partial segments of waveform data in an electrocardiogram as input information and employing peak information indicating whether or not a predetermined type of peak related to analysis of the electrocardiogram is present in the waveform data as output information, an analysis waveform acquisition unit 11C that acquires waveform data in an electrocardiogram for analysis, an analysis waveform select and extract unit 11D that selects and extracts waveform data of a predetermined first period from the waveform data acquired by the analysis waveform acquisition unit 11C as analysis divided waveform data while each time shifting a predetermined shift period that is a shorter period than the first period, and a derivation unit 11E that derives the peak information by inputting the analysis divided waveform data selected and extracted by the analysis waveform select and extract unit 11D into the peak estimation model 13C. The peak information obtained by the peak estimation model 13C is accordingly employed, enabling a contribution toward determining whether or not a particular type of arrhythmia has occurred.

Moreover, one exemplary embodiment also includes a training waveform acquisition unit 11A that acquires waveform data in electrocardiograms for training, and a training waveform select and extract unit 11B that selects and extracts waveform data of the first period from the waveform data acquired by the training waveform acquisition unit 11A as training divided waveform data. The peak estimation model 13C is a model trained by machine learning employing the training divided waveform data selected and extracted by the training waveform select and extract unit 11B as input information and employing the peak information corresponding to the training divided waveform data as output information. This thereby enables a contribution toward determining whether or not a particular type of arrhythmia has occurred with higher accuracy than cases in which waveform data selected and extracted at a different period to the waveform data during operation of the peak estimation model 13C is employed for machine learning.

Moreover, in one exemplary embodiment, the predetermined type includes at least one type from out of a first type indicting normal or atrial premature complex, a second type indicating atrial fibrillation, and a third type indicating premature ventricular contraction. This thereby enables a contribution to discovering an arrhythmia of these employed types.

Moreover, one exemplary embodiment further includes a segmentation estimation model 13D that employs waveform data of partial segments of waveform data in an electrocardiogram as input information and employs segment type information indicating whether or not a segment corresponding to the waveform data is one segment of predetermined types of segment as output information, a second analysis waveform select and extract unit 11F that selects and extracts waveform data of a second period that is a longer period than the first period from waveform data acquired by the analysis waveform acquisition unit 11C as second analysis divided waveform data, a second derivation unit 11G that derives the segment type information by inputting the second analysis divided waveform data selected and extracted by the second analysis waveform select and extract unit 11F into the segmentation estimation model 13D, and an estimation unit 11H that estimates a condition indicated by the electrocardiogram for analysis by synthesizing the peak information derived by the derivation unit 11E together with the segment type information derived by the second derivation unit 11G. This thereby enables a contribution toward determining whether or not a particular type of arrhythmia has occurred with higher accuracy than cases in which the segmentation estimation model 13D is not employed.

Moreover, in one exemplary embodiment, the predetermined types of segment include at least two segments from out of a normal segment that is a segment that is normal, an atrial fibrillation segment that is a segment with an atrial fibrillation, and a non-analysis segment that is a segment not for analysis. This thereby enables a contribution to identifying these employed segments.

Moreover, one exemplary embodiment is an electrocardiogram analysis assistance device employing a peak estimation model 13C employing waveform data of partial segments of waveform data in an electrocardiogram as input information and employing peak information indicating whether or not a predetermined type of peak related to analysis of the electrocardiogram is present in the waveform data as output information, an analysis waveform acquisition unit 11C that acquires waveform data in an electrocardiogram for analysis, an analysis waveform select and extract unit 11D that selects and extracts waveform data of a predetermined first period from the waveform data acquired by the analysis waveform acquisition unit 11C as analysis divided waveform data, an intermediate information acquisition unit 11I that acquires intermediate information generated in a middle layer of the peak estimation model 13C and indicating a shape feature of a peak contained in the analysis divided waveform data by inputting the analysis divided waveform data selected and extracted by the analysis waveform select and extract unit 11D into the peak estimation model 13C, and a classification unit 1 1J that classifies a type of the waveform of the electrocardiogram for analysis using the intermediate information acquired by the intermediate information acquisition unit 111. This thereby enables a contribution to identifying a type of the waveform of the electrocardiogram for analysis.

Moreover, in one exemplary embodiment, the above types of waveform include at least two types from out of the standard type, the type having peaked T waves taller than the standard type, the type having wide QRS waves wider than the standard type, or the other-type therefrom. This thereby enables a contribution to identifying these employed types. Note that the type having peaked T waves taller than the standard type is a type corresponding to hyperkalemia, and so including this type in the above waveform types enables the possibility of there being a serum electrolyte imbalance to be ascertained. The type having wide QRS waves wider than the standard type is a type corresponding to a bundle branch block, and so including this type in the above waveform types enables the possibility of severe myocardial disease or the like to be ascertained.

Furthermore, one exemplary embodiment performs the above classification by clustering the intermediate information. This thereby enables a contribution to identifying the waveform type of the electrocardiogram for analysis more appropriately than cases in which there is no clustering of intermediate information.

Note that although in the above exemplary embodiment a case has been described in which the electrocardiographic condition information and the classification result information are presented by displaying on a display section, there is no limitation thereto. For example, an embodiment may be adopted in which the electrocardiographic condition information and the classification result information is presented by speech using the voice output section 19, or an embodiment may be adopted in which the electrocardiographic condition information and the classification result information is presented by being printed using an image forming device such as a printer.

Moreover, although in the above exemplary embodiment a case has been described in which the electrocardiogram analysis assistance device of the present disclosure is configured by device having a standalone configuration, there is no limitation thereto. For example, an embodiment may be adopted in which the electrocardiogram analysis assistance device according to the present disclosure is configured by a system employing plural devices including a server device such as a cloud server and a terminal device. In such cases, for example, an electrocardiogram of a user may be measured with the terminal device and waveform data obtained by this measurement transmitted to the server device, with the electrocardiogram analysis assistance processing illustrated as an example in Fig. 10 being executed using the waveform data received by the server device as the analysis target. An embodiment may be given as an example in which the electrocardiographic condition information and the classification result information obtained in this manner is then transmitted to the terminal device, and this information presented using the terminal device.

Moreover, in the above exemplary embodiment, for example, each of the following types of processor may be employed as hardware structure of a processing unit that executes each of the processing of the training waveform acquisition unit 11A, the training waveform select and extract unit11B, the analysis waveform acquisition unit 11C, the analysis waveform select and extract unit 11D, the derivation unit 11E, the second analysis waveform select and extract unit 11F, the second derivation unit 11G, the estimation unit 11H, the intermediate information acquisition unit 11I, and the classification unit 1 1J. Such types of processor include, in addition to a CPU that is a general processor that executes software (a program) to function as a processing unit as described above, a programmable logic device (PLD) that is a processor that allows circuit configuration to be modified post-manufacture, such as a fieldprogrammable gate array (FPGA), and dedicated electric circuits, these being processors including a circuit configuration custom-designed to execute specific processing such as an application specific integrated circuit (ASIC).

The processing unit may be configured from any one of these various types of processor, or may configured by a combination of two or more of the same type or different types of processor (such as a combination of plural FPGAs, or a combination of a CPU and an FPGA). The processing unit may also be configured as a single processor.

Examples in which the processing unit is configured by a single processor include, firstly, a configuration of a single processor combining one or more CPU and software as typified by a computer such as a client or server, in an embodiment in which this processor functions as the processing unit. Secondly, there is an embodiment using a processor to implement the functions of an entire system including the processing unit with a single integrated circuit (IC) chip as typified by a system on chip (SOC) or the like. In this manner the processing unit is configured using one or more of these types of processor as a hardware structure.

Furthermore, more specifically, circuitry combining circuit elements such as semiconductor elements may be employed as an example of a hardware structure of these various types of processor.

### Supplements

The following supplements related to the above exemplary embodiments are furthermore disclosed.

### Supplement 1

An electrocardiogram analysis assistance device including a processor,
the processor executing processing including:
acquiring waveform data in an electrocardiogram for analysis;
selecting and extracting waveform data of a predetermined first period from the acquired waveform data as analysis divided waveform data, while each time shifting a predetermined shift period that is a shorter period than the first period; and
deriving the peak information by inputting the selected and extracted analysis divided waveform data into a peak estimation model employing waveform data of partial segments of waveform data in an electrocardiogram as input information and employing peak information indicating whether or not a predetermined type of peak related to analysis of the electrocardiogram is present in the waveform data as output information.

### Supplement 2

A non-transitory storage medium stored with a program that causes a computer to execute processing using a peak estimation model employing waveform data of partial segments of waveform data in an electrocardiogram as input information and employing peak information indicating whether or not a predetermined type of peak related to analysis of the electrocardiogram is present in the waveform data as output information. The processing including:
acquiring waveform data in an electrocardiogram for analysis;
selecting and extracting waveform data of a predetermined first period from the acquired waveform data as analysis divided waveform data, while each time shifting a predetermined shift period that is a shorter period than the first period; and
deriving the peak information by inputting the selected and extracted analysis divided waveform data into the peak estimation model.

The entire content of the disclosure of Japanese Patent Application No. 2021-050381 filed on March 24, 2021 is incorporated by reference in the present specification.

## Claims

1. An electrocardiogram analysis assistance device comprising:
a peak estimation model employing waveform data of partial segments of waveform data in an electrocardiogram as input information and employing peak information indicating whether or not a predetermined type of peak related to analysis of the electrocardiogram is present in the waveform data as output information;
an analysis waveform acquisition unit that acquires waveform data in an electrocardiogram for analysis;
an analysis waveform select and extract unit that selects and extracts waveform data of a predetermined first period from the waveform data acquired by the analysis waveform acquisition unit as analysis divided waveform data, while each time shifting a predetermined shift period that is a shorter period than the first period; and
a derivation unit that derives the peak information by inputting the analysis divided waveform data selected and extracted by the analysis waveform select and extract unit into the peak estimation model.

2. The electrocardiogram analysis assistance device of claim 1, further comprising:
a training waveform acquisition unit that acquires waveform data in electrocardiograms for training; and
a training waveform select and extract unit that selects and extracts waveform data of the first period from the waveform data acquired by the training waveform acquisition unit as training divided waveform data, wherein
the peak estimation model is a model trained by machine learning employing the training divided waveform data selected and extracted by the training waveform select and extract unit as input information and employing the peak information corresponding to the training divided waveform data as output information.

3. The electrocardiogram analysis assistance device of claim 1 or claim 2, wherein:
the predetermined type includes at least one type from out of a first type indicting normal or atrial premature complex, a second type indicating atrial fibrillation, and a third type indicating premature ventricular contraction.

4. The electrocardiogram analysis assistance device of any one of claim 1 to claim 3, further comprising:
a segmentation estimation model that employs waveform data of partial segments of waveform data in an electrocardiogram as input information and employs segment type information indicating whether or not a segment corresponding to the waveform data is one segment of predetermined types of segment as output information;
a second analysis waveform select and extract unit that selects and extracts waveform data of a second period that is a longer period than the first period from waveform data acquired by the analysis waveform acquisition unit as second analysis divided waveform data;
a second derivation unit that derives the segment type information by inputting the second analysis divided waveform data selected and extracted by the second analysis waveform select and extract unit into the segmentation estimation model; and
an estimation unit that estimates a condition indicated by the electrocardiogram for analysis by synthesizing the peak information derived by the derivation unit together with the segment type information derived by the second derivation unit.

5. The electrocardiogram analysis assistance device of claim 4, wherein the predetermined types of segment include at least two segments from out of a normal segment that is a segment that is normal, an atrial fibrillation segment that is a segment with an atrial fibrillation, and a non-analysis segment that is a segment not for analysis.

6. A program that causes a computer to execute processing using a peak estimation model employing waveform data of partial segments of waveform data in an electrocardiogram as input information and employing peak information indicating whether or not a predetermined type of peak related to analysis of the electrocardiogram is present in the waveform data as output information, the processing comprising:
acquiring waveform data in an electrocardiogram for analysis;
selecting and extracting waveform data of a predetermined first period from the acquired waveform data as analysis divided waveform data, while each time shifting a predetermined shift period that is a shorter period than the first period; and
deriving the peak information by inputting the selected and extracted analysis divided waveform data into the peak estimation model.

7. An electrocardiogram analysis assistance method using a peak estimation model employing waveform data of partial segments of waveform data in an electrocardiogram as input information and employing peak information indicating whether or not a predetermined type of peak related to analysis of the electrocardiogram is present in the waveform data as output information, the electrocardiogram analysis assistance method comprising:
acquiring waveform data in an electrocardiogram for analysis;
selecting and extracting waveform data of a predetermined first period from the acquired waveform data as analysis divided waveform data, while each time shifting a predetermined shift period that is a shorter period than the first period; and
deriving the peak information by inputting the selected and extracted analysis divided waveform data into the peak estimation model.

8. An electrocardiogram analysis assistance system comprising:
the electrocardiogram analysis assistance device of any one of claim 1 to claim 5; and
a terminal device that transmits waveform data in an electrocardiogram for analysis to the electrocardiogram analysis assistance device and that receives and presents information obtained by the electrocardiogram analysis assistance device in response to the transmission of the waveform data.

9. A peak estimation model generation method comprising:
acquiring first training waveform data in electrocardiograms for training;
selecting and extracting waveform data of a predetermined first period from the acquired first training waveform data as first training divided waveform data; and
generating a peak estimation model by performing machine learning employing the selected and extracted first training divided waveform data as input information and employing peak information corresponding to the first training divided waveform data and indicating whether or not a predetermined type of peak related to electrocardiogram analysis is present as output information.

10. A segmentation estimation model generation method comprising:
acquiring second training waveform data in electrocardiograms for training;
selecting and extracting waveform data of a second period that is a longer period than the predetermined first period from the acquired second training waveform data as second training divided waveform data; and
generating a segmentation estimation model by performing machine learning employing the selected and extracted second training divided waveform data as input information and employing segment type information indicating whether or not a segment corresponding to the second training divided waveform data is a segment out of predetermined types of segment as output information.
